# EUROPEAN PATENT SPECIFICATION

(11) **EP 0 514 509 B1**
(45) Date of publication and mention of the grant of the patent: **20.09.2000**
(21) Application number: 91920356.2
(22) Date of filing: 28.11.1991
(51) Int. Cl.: G01N 33/569

(54) **COMPOSITION AND ITS PREPARATION PROCESS USING ANTIGEN CONJUGATED TO ENZYMATIC ACTIVITY FOR IMMUNOLOGICAL DIAGNOSIS AND CHAGAS' DISEASE IMMUNOLOGICAL DIAGNOSIS KITS, FOR INDIVIDUAL AND EPIDEMIOLOGICAL APPLICATION, BASED ON THAT COMPOSITION**
ZUSAMMENSETZUNG ZUR IMMUNOLOGISCHEN ANALYSE UND IHR HERSTELLUNGSVERFAHREN UNTER VERWENDUNG VON ANTIGEN, SOWIE IMMUNOLOGISCHE DIAGNOSEKITS FÜR DIE CHAGAS-KRANKHEIT
COMPOSITION COMPRENANT DES ANTIGENES CONJUGUES A UNE ACTIVITE ENZYMATIQUE, ET DESTINEE AUX NECESSAIRES A USAGE PRIVE ET EPIDEMIOLOGIQUE DE DIAGNOSTIC IMMUNOLOGIQUE, NOTAMMENT DE LA MALADIE DE CHAGAS, ET PROCEDE DE PREPARATION DE LADITE COMPOSITION

(30) Priority: 28.11.1990 BR 9006039
(43) Date of publication of application: 25.11.1992
(73) Proprietor: FUNDACAO OSWALDO CRUZ (FIOCRUZ) - SUPERINTENDENCIA DE PLANEJAMENTO, BR-21040 Rio de Janeiro, RJ (BR)
(72) Inventor: CERQUEIRA DE ALMEIDA, Elza Carmen, 22740-Rio de Janeiro, RJ (BR); KRIEGER, Marco Aurelio, Rua Desembargador Antonio, Ilha do Governador, 21902 Rio de Janeiro (BR); GOLDENBERG, Samuel, 22471-Rio de Janeiro, RJ (BR)
(74) Representative: Derambure, Christian
(86) International application number: BR9100027
(87) International publication number: WO9209895

(56) References cited:
- EP-A- 0 135 108
- EP-A- 0 138 101
- EP-A- 0 273 555
- WO-A-91/15584
- US-A- 3 911 097
- US-A- 3 993 743
- US-A- 4 609 630
- CHEMICAL ABSTRACTS, Volume 99, No. 19, issued 7 November 1983 (Columbus, Ohio, US), E. BITTENCOURT et al.: "Immunoenzymic assays conducted with resin-treated nylon fabrics", see page 455, column 1, the Abstract No. 156455g, J. Polym. Sci., Polym. Lett. Ed. 1983, 21(9), 717-21 (Eng).
- CHEMICAL ABSTRACTS, Volume 104, No. 11, issued 17 March 1986 (Columbus, Ohio, US), M. SCHECHTER et al., "Further evaluation of lectin affinity purified glycoprotein (GP90) in the enzyme linked immunosorbent assay (ELISA) for diagnosis of Trypanosoma cruzil infection", see page 490, column 2, the Abstract No. 86562k, Trans. R. Soc. Trop. Med. Hyg. 1985, 79(5), 637-40 (Eng).
- PRINCIPLES OF COMPETITIVE PROTEIN-BINDING ASSAYS, 2nd ed., W.D. Odell et al., (editors), J. Wiley & Sons; L. WIDE, chapter 13
- TROPICAL DISEASE RESEARCH, Progress 1991-92, WHO, Geneva (CH); A. MONCAYO
- ENZYME IMMUNOASSAY, publisher CRC Press (1981), p. 194

## Description

### BACKGROUND OF THE INVENTION

This patent request refers to the use of antigens conjugated to an enzimatic activity (detectable by color reaction), in diagnostic assays for Chagas' disease and other infectious diseases.

The methods generally used for the serological diagnosis of infectious diseases, rely on the presence of antibodies against the pathogen, in the sera of the infected patient. But these techniques can present some limitations in their specificity when crude antigen preparations are used. On the other hand, the use of purified antigens can present problems in terms of cost and large scale aplicability. However, the development of recombinant DNA techniques paved the way to the large scale obtention of purified antigens with lower cost and safer conditions. Accordingly, our work with Trypanosoma cruzi ( the ethiological agent of Chagas' disease), resulted in the cloning and expression in bacteria of antigens from the parasite that are specifically recognized by chagasic sera. T.cruzi DNA was cloned in E.coli using the expression vector lambda gtii, and the recombinant clones were screened with a pool of human chagasic sera. Twelve recombinant clones were purified to homogeneity and from these two were selected on the basis of the specificity of their reactivity with chagasic sera. Partial nucleotide sequence of these clones showed that one of them (Ag#1) encodes an antigen composed of at least 23 repetitions of a 14 aminoacids motif : Valine (or alanine, alanine, glutamic acid, alanine (or threonine), glutamic acid, lysine, glutamine, lysine (or arginine), alanine alanine, glutamic acid, alanine (or serine), threonine (or methionine or alanine) and lysine, whereas the other (Ag#2) encodes an antigen containing at least 14 repetitions of a 68 aminoacids repetitive epitope: Methionine glutamic acid glutamine, glutamic acid, arginine, arginine, glutamine leucine, leucine, glutamic acid, lysine, aspartic acid proline, arginine, arginine, asparagine, alanine, lysine glutamic acid, isoleucine, alanine, alanine, leucine glutamic acid, glutamic acid, serine, methionine asparagine, alanine, arginine, alanine, glutamine glutamic acid, leucine, alanine, arginine, glutamic acid lysine, lysine, leucine, arginine, aspartic acid arginine, alanine, phenylalanine, leucine, aspartic acid glutamine, lysine, proline, glutamic acid, arginine, valine, proline, leucine, alanine, aspartic acid, valine, proline, leucine, aspartic acid, aspartic acid, aspartic acid, serine, aspartic acid, phenylalanine, valine and alanine. The recombinant antigens Ag#1 and Ag#2, as well as a synthetic peptide corresponding to Ag#1, were tested in the immunological diagnosis of Chagas Disease, using the methods of ELISA, dot-blot, western blot and agglutination. The results obtained showed that the aforementioned antigens provide an adequate and specific diagnosis for Chagas Disease.

The ELISA test (enzyme linked immunoenzimatic assay) is frequently used for serological diagnosis. This method allows the identification and quantification of antigens or antibodies in biological fluids. The conventional ELISA consists in the detection of the complex antibody-antigen by a second antibody (against the antibody that reacts with the antigen) conjugated to an enzimatic activity (peroxidase, alkaline phosphatase and others).

Alternatively, the immune-complex can be also detected using Staphylococcus aureus protein-A or protein-G conjugated to the enzimatic activity. However, this conventional ELISA does not detec low titer antibodies, resulting in the failure to distinguish among positive and negative sera. In the case of the use of recombinant antigens, this problem can be amplified in virtue of the cross-reactivity with bacterial antigens that can eventually contaminate the preparation.

A method based on the detection of highly specific antibodies (irrespective to their titre) should avoid the limitations of the ELISA test described above. This can be performed by using the antigen to sensitize the ELISA support and to develop the immune-complex through its conjugation to the enzymatic activity or radioactive labelling.

In Enzyme-Immunoassay, pub. by CRC Press (1981), page 194 (Appendix 4), it is described an example of the indirect microplate ELISA (for the detection of antibody to thyroglobulin). This is a competitive binding assay in which both the analyte (antibody contained in test sera) and Enzyme-labeled antihuman immunoglobulin compete the same binding site and the analyte is indirectly measured. To obtain high sensitivity with this method it is essencial that almost all the labeled binding reagent be bound when the incubation is interrupted and that the dissociation rate constant be low for the analyte-binding reagent reaction. The dissociation of this complex is influenced by the amount of solid phase ligand, and, the kinetics of all these reactions are of importance in determining the sensitivity of the system (Odell et al., Eds., Principles of Competitive Protein Binding Assays, John Wiley & Sons, New York, 1983, pp. 243-254).

The Patent Application EP 0273 555 A2 describes an antigenic material for a Chagas'disease detection system. The antigenic material is a 70 kd polypeptide having an amino acid sequence as forth in Figure 4, which is recognized by all of the chagasic sera test (2 Argentinian, 1 Columbian and 1 Brazilian), but not by Leishmania sera (page 3, column 4). On page 7, column 12, it is described that different concentrations of antigen and various dilutions of sera are used in an ELISA test system for Chagas'disease and the results of this series of assays provides the optimal as well minimal levels of sensitivity using the synthesized 70 kd antigen. Such a method has three important limitations, the first being the necessity to dilute the sera, the second being the use of a competitive binding assay, and, the third being the absence of data showing the sensitivity and specificity of the antigen.

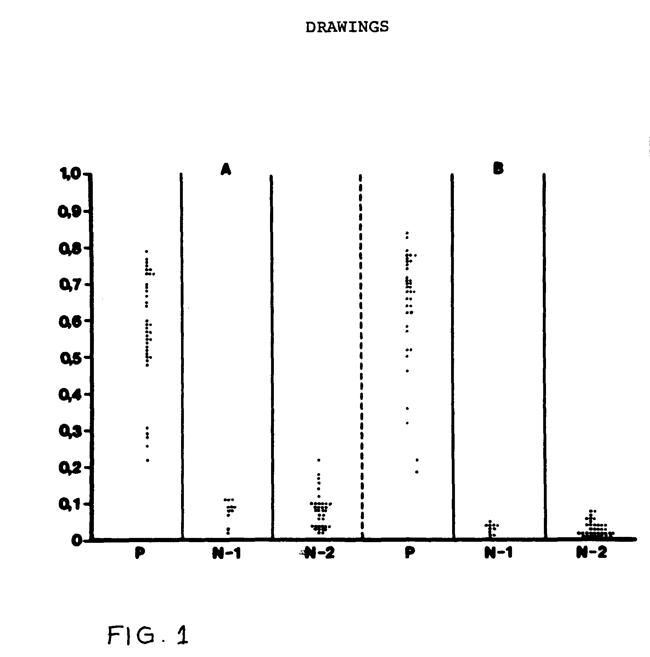

This patent application refers to the development of a direct ELISA and its use in the diagnosis of Chagas' disease. This method relies on the use of the antigen for sensitizing the plate and for the detection of the immune-complexes. The method, which is graphically represented in the scheme of Figure 1, is fast and allows the visual reading of the result.

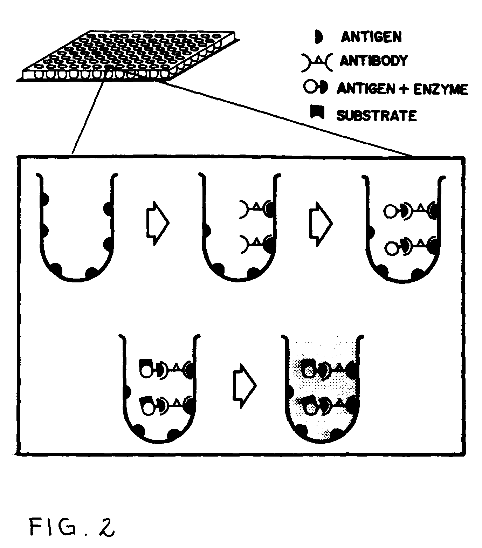

Some results using the direct ELISA and the comparison with a conventional ELISA are described below. The results presented in figure 2 compare the reactivity of human chagasic sera (lane P), non-chagasic sera from endemic areas (lane N-1) and non-chagasic sera from blood bank (N-2) using a conventional ELISA (Fig. 1-A) and the direct ELISA (Fig. 1-B). It can be observed that the cut-off line is much more clear in the case of the direct than in the case of the indirect ELISA. The direct ELISA is such more specific and sensible than the conventional ELISA since no false positive or false negative results were observed using this method.

### DESCRIPTION OF THE INVENTION

The procedures of the method are as follows. The antigen or antigenic mixture, in a concentration ranging from 0.01 to 1 mg/ml, depending on the antigen, is immobilized in the solid support. The antigen is diluted in 50mM carbonate buffer, phosphate buffer or any other suitable buffer for immobilization to the support, which can be polycarbonate, polyallomer, polypropilene, polyvinyl, nylon and nitrocellulose. The solid phase sensitized with the antigen is washed with phosphate buffered saline pH 7.0-7.5 containinig 0,3% detergent, and dried out. The sera to be analysed are applied onto the support containing the antigen and the ensemble is incubated at 25-37 C for times varying from 30 minutes to 120 minutes.

Following this, the solid phase is washed with phosphate buffered saline or any other suitable buffer.

The immune-complexes are developed with the total or fractions of the antigenic mixture used to sensitize the support, conjugated to an enzimatic activity or radioactivelly labelled. The conjugated antigen is diluted in phosphate buffered saline, or any suitable buffer, containing a blocking agent such as gelatin, bovine sera, BSA, milk, etc., in order to avoid unespecific binding to the support. After incubation at 37° C, the immune-complexes are developed according to the enzimatic reaction suitable for the conjugated enzyme or radioactive labelling.

Two kits for the diagnosis of Chagas' disease were developed based on the procedures described above: a direct ELISA kit and a kit using a membrane (nylon or nitrocellulose) as support. The Direct ELISA kit is composed of a micro-titre plate coated with the antigens (described in the International Application under the Patent Cooperation Treaty PCT/BR 91/00003, Brazilian Application number 9001479) in a concentration of 0.1mg/ml, a flask containing the conjugated antigen, three tubes containing control sera that consist of negative, positive and cut-off sera, a flask with washing buffer (buffered saline solution, pH7.5, containing 0.3% Tween® -20), washing buffer containing foetal bovine sera 5% and the solutions necessary for developing the enzimatic reaction. The kit also contains an explanatory note on how to proceed. Briefly, the sera to be tested (undiluted or diluted until 1:200) in a volume of 50µl are put in the well and incubated for 30-60 minutes at 37° C and then removed. The wells are washed three times with washing solution as described above and then, the antigen conjugated to the enzymatic activity diluted 1:1000 is added in a final volume of 50µl and incubated at 37° C for 60 minutes. The wells are washed three times with washing buffer and then the substrate of the enzyme is added (3,3', 5,5'- tetramethyl benzidine 0,01 mg/ml in DMSO and 0,3% hydrogen peroxide in the case of peroxidase). Following 10 minutes of incubation at room temperature, the reaction is stopped by the addition of 4M sulphuric acid. The results can be read visually or in the spectrophotometer by the absorbance at 450nm.

In the case of the kit using a solid support (nitrocellulose, nylon, polyallomer, polycarbonate, polyvinyl, etc.), the antigens are spread onto the membrane and their position is signaled. The strips are then placed inside a tube that is the reaction vessel. The kit contains 20 tubes with strips, the conjugate solution, control sera and washing buffer consisting of phosphate buffered saline solution (pH 7.5) containing 0.3% Tween® -20. In addition, the kit contains a dilution solution (washing buffer containing 5% bovine serum) and the reagents necessary for developing the enzymatic reaction, as described above. The serum sample to be tested is diluted 1:100 in washing solution and 2ml of this solution are put inside the reaction tube containing the membrane.

After incubation at. 37° C for 60 minutes, the serum is removed and the strip is washed three times with 2ml of washing solution. Following this, the conjugate in a final volume of 2ml and in a dilution of 1:1000 is added to the reaction vessel containing the strip, and incubated for additional 60 minutes at 37° C. The conjugate is removed, the strip is washed twice with washing buffer and the substrate of the enzyme is added. The reaction proceeds for 10 minutes and is then stopped with water. The result of the reaction is determined by the intensity of the color respective to the controls.

These kits are stable for at least six months at 4° C and for one month at 37° C. The conjugate is kept in physiological saline solution containing 20% glycerol and proteinase inhibitors.

## Claims

1. A kit for the immunological diagnosis of Chagas'disease comprising the following:
a support of a material selected from the group consisting of polycarbonate, polyallomer, polypropylene, polyvinyl, nylon and nitrocellulose;
and reactants, including the following:
(i) a 50 mM carbonate buffer containing 0.01 to 1.0 mg/ml of a mixture of Antigen #1 and Antigen #2;
(ii) a washing buffer of a phosphate buffered saline (PBS) pH 7.0 with 0.3% of detergent;
(iii) an enzymatic activity coupled to the Antigen #1 and the Antigen #2 in a solution comprising enzyme-conjugated Antigen #1 and enzyme-conjugated Antigen #2 for development of an immunocomplex;
(iv) a dilution buffer consisting of the washing buffer containing 5% of bovine sera for the dilution of the conjugated solution;
(v) a substrate solution, to develop the enzymatic activity;
(vi) a stop solution, to block the enzymatic activity;
(vii) a standard sera containing a positive, negative and cut off serum,
Antigen #1 having the following aminoacid sequence: Valine (or alanine), alanine, glutamic acid, alanine (or threonine), glutamic acid, lysine, glutamine, lysine (or arginine), alanine, alanine, glutamic acid, alanine (or serine), threonine (or methionine or alanine) and lysine and Antigen #2 having the following aminoacid sequence: Methionine, glutamic acid glutamine, glutamic acid, arginine, arginine, glutamine leucine, leucine, glutamic acid, lysine, aspartic acid proline, arginine, arginine, asparagine, alanine, lysine glutamic acid, isoleucine, alanine, alanine, leucine glutamic acid, glutamic acid, serine, methionine asparagine, alanine, arginine, alanine, glutamine glutamic acid, leucine, alanine, arginine, glutamic acid lysine, lysine, leucine, arginine, aspartic, acid arginine, alanine, phenylalanine, leucine, aspartic acid, glutamine, lysine, proline, glutamic acid, arginine, valine, proline, leucine, alanine, aspartic acid, valine, proline, leucine, aspartic acid, aspartic acid, aspartic acid, serine, aspartic acid, phenylalanine, valine and alanine.

2. The kit according to claim 1 wherein said solution comprising enzyme-conjugated Antigen #1 and enzyme-conjugated Antigen #2 has been prepared by the steps comprising the following:
(i) treatment of the enzyme with 0,02M metaperiodate following dialysis against a 1mM acetate buffer pH 4.4;
(ii) addition of the treated enzyme to the purified mixture of Antigen #1 and Antigen #2;
(iii) addition of a 0.0015M sodium carbonate solution, pH 9.5, to the solution from step (ii) and incubation at room temperature for 30 to 120 minutes;
(iv) addition of a 0.2 mg/ml solution borohydrate solution to the solution resulting from step (iii), at 4°C for two hours; and
(v) dialysis of the solution resulting from step (iv) against a phosphate buffered saline, pH 7.2, antigen #1 and Antigen #2 being the Antigen set forth in claim 1.

3. The kit according to claim 1 wherein the enzymatic activity is selected from the group consisting of peroxidase, alkaline phosphatase and urease.

4. The kit according to claim 3 wherein the substrate solution is a 0.01M citrate or phosphate buffer containing 1 mg of 3',3',5,5' tetramethylbenzydine in 1 ml DMSO and 0.3% hydrogen peroxide, when the enzymatic activity is peroxidase.

5. The kit according to claim 1 wherein the stop solution is a 4M sulfuric acid solution, when the enzymatic activity is peroxidase.

6. The kit according to claim 1 wherein the support is a multi well plate for use in the direct ELISA.

7. The kit according to claim 1 wherein the support is a membrane for use in the direct strip test.

8. The kit according to claim 1 wherein the immunocomplex is developed with the use of the Antigen #1 and Antigen #2 (as set forth in Claim 1) conjugated to the enzymatic activity.

9. A method for the immunological diagnosis of Chagas'disease comprising the following steps:
(i) sensitizing a support of a material selected from the group consisting of polycarbonate, polyallomer, polypropylene, polyvinyl, nylon or nitrocellulose with a 50mM carbonate buffer containing 0.01 to 1.0 mg/ml of a mixture of the Antigens set forth in claim 1
(ii) washing the sensitized support with a washing buffer of a phosphate buffered saline (PBS) pH 7.0 with 0.3% of detergent;
(iii) adding to the ensemble of the step (ii) the serum to be tested and incubating the ensemble for a sufficient time to permit the specific binding between the antigens and antibodies to *Trypanosoma cruzi*.
(iv) washing the ensemble which contains the serum with the same buffer solution used in the step (ii);
(v) preparing a solution comprising enzymes-conjugated with the Antigens set forth in claim 1, said enzymes having an enzymatic activity;
(vi) diluting the solution comprising enzyme-conjugated Antigen #1 and enzyme-conjugated Antigen #2 as prepared in (v) to block nonspecific binding sites, said diluting solution consisting of the washing buffer which contains 5% bovine sera;
(vii) adding to the ensemble of step (iv) the solution of the step (vi) and incubating for a sufficient time to permit the interaction between the antibodies to *Trypanosoma cruzi* and the antigens having an enzymatic activity;
(viii) effecting the development of the immunocomplex by adding to the ensemble of step (vii) the substrate solution and then incubating for a sufficient time to permit the interaction between the enzyme and the substrate;
(ix) blocking the enzymatic activity by addition of a stop solution; and
(x) evaluating the results of the enzymatic activity in relation to a standard serum.

10. The method according to claim 9 wherein the step (iii) is carried out at room temperature between 25°C to 37°C, in a period of time from 30 minutes to 2 hours.

11. The method according to claim 9 wherein the step (vii) is carried out at room temperature between 25°C to 37°C, during 30 minutes.

12. The method according to claim 9 wherein the step (viii) is carried out at room temperature between 25°C to 37°C, during 10 minutes.

## Patentansprüche

1. Reagenziensatz für die Immundiagnose der Chagas-Krankheit, bestehend aus
einem Träger aus einem Material das von der Gruppe ausgewählt wird, die aus Polycarbonat, Polyallomer, Polypropylen, Polyvinyl, Nylon und Nitrocellulose besteht;
und aus folgenden Reagenzien:
(i) ein 50 mM Carbonatpuffer welcher 0,01 bis 1,0 mg/ml einer Mischung von Antigen #1 und Antigen #2 enthält;
(ii) ein Auswaschpuffer aus einer phosphatgepufferten Salzlösung (PBS) bei pH 7,0 mit 0,3% Detergen;
(iii) eine mit dem Antigen #1 und dem Antigen #2 gekoppelte enzymatische Aktivität, in einer Lösung, die das enzymkonjugierte Antigen #1 und das enzymkonjugierte #2 zur Bildung eines Immunkomplexes enthält;
(iv) ein Verdünnungspuffer zur Verdünnung der konjungierten Lösung der aus dem Auswaschpuffer mit 5% Rinderserum besteht;
(v) eine Substratlösung um die enzymatische Aktivität zu entwickeln;
(vi) eine Stoplösung um die enzymatische Aktivität zu hemmen;
(vii) Standardsera mit einem positiven, negativen und Sperrserum,
wobei Antigen #1 folgende Aminosäurensequenz aufweist: Valin (oder Alanin), Alanin, glutamische Säure, Alanin (oder Threonin), glutamiche Säure, Lysin, Glutamin, Lysin (oder Arginin), Alanin, Alanin, glutamische Säure, Alanin (oder Serin), Threonin (oder Methionin oder Alanin) und Lysin und wobei Antigen #2 folgende Aminosäurensequenz aufweist: Methionin, glutamische Säure, Glutamin, glutamische Säure, Arginin, Arginin, Glutamin, Leucin, Leucin, glutamische Säure, Lysin, Asparginsäure, Prolin, Arginin, Arginin, Aspargin, Alanin, Lysin, glutamische Säure, Isoleucin, Alanin, Alanin, Leucin, glutamische Säure, glutamische Säure, Serin, Methionin, Aspargin, Alanin, Arginin, Alanin, Glutamin, glutamische Säure, Leucin, Alanin, Arginin, glutamische Säure, Lysin, Lysin, Leucin, Arginin, Asparginsäure, Arginin, Alanin, Phenylalanin, Leucin, Asparginsäure, Glutamin, Lysin, Prolin, glutamische Säure, Arginin, Valin, Prolin, Leucin, Alanin, Asparginsäure, Valin, Prolin, Leucin, Asparginsäure, Asparginsäure, Asparginsäure, Serin, Asparginsäure, Phenylalanin, Valin und Alanin.

2. Reagenziensatz gemäß Anspruch 1, wobei die genannte Lösung, die das enzymkonjugierte Antigen #1 und das enzymkonjugierte Antigen #2 enthält, durch folgende Verfahrensschritte hergestellt wurde:
(i) Behandlung des Enzyms mit 0,02M Metaperiodat nach einer Dialyse gegen einen 1 mM Acetatpuffer bei pH 4,4;
(ii) Zugabe des behandelten Enzyms zu der gereinigten Mischung aus Antigen #1 und Antigen #2;
(iii) Zugabe einer 0,0015M Natriumcarbonatlösung, bei pH 9,5, zu der Lösung von Schritt (ii) und Inkubation bei Raumtemperatur während 30 bis 120 Minuten;
(iv) Zugabe einer 0,2 mg/ml Borhydratlösung zu der aus Schritt (iii) erhaltenen Lösung, bei 4°C, während zwei Stunden; und
(v) Dialyse der Lösung aus Schritt (iv) gegen eine phosphatgepufferte Salzlösung bei pH 7,2, wobei Antigen #1 und Antigen #2 die in Anspruch 1 aufgeführten Antigene sind.

3. Reagenziensatz gemäß Anspruch 1, wobei die enzymatische Aktivität aus der Gruppe ausgewählt wird, die aus Peroxidase, basische Phosphatase und Urease besteht.

4. Reagenziensatz gemäß Anspruch 3, wobei die Substratlösung ein 0,01M Zitrat oder Phosphatpuffer ist, mit einem mg 3, 3', 5, 5' Tetramethylbenzidin in 1 ml DMSO und 0,3% Wasserstoffperoxid, wenn die enzymatische Aktivität Peroxidase ist.

5. Reagenziensatz gemäß Anspruch 1, wobei die Stoplösung eine 4M Schwefelsäurelösung ist, wenn die enzymatische Aktivität Peroxidase ist.

6. Reagenziensatz gemäß Anspruch 1, wobei der Träger eine Multibrunnenplatte für Direkt-ELISA-Messungen ist.

7. Reagenziensatz gemäß Anspruch 1, wobei der Träger eine Membrane für Direkt-Strip-Tests ist.

8. Reagenziensatz gemäß Anspruch 1, wobei der Immunkomplex mit Hilfe des Antigen #1 und des Antigen #2 (gemäß Anspruch 1), konjungiert mit der enzymatischen Aktivität, entwickelt wird.

9. Verfahren für die Immundiagnose der Chagaskrankheit mit folgenden Schritten:
(i) Sensibilisierung eines Trägers aus einem Material, das aus der Gruppe, die aus Polycarbonat, Polyallomer, Polypropylen, Polyvinyl, Nylon oder Nitrocellulose besteht, ausgewählt ist, mit einem 50mM Carbonatpuffer, der 0,01 bis 1,0 mg/ml einer Mischung der Antigene gemäß Anspruch 1 enthält;
(ii) Auswaschen des sensibilisierten Trägers mit einem Auswaschpuffer eine phosphatgepufferte Salzlösung (PBS) bei pH 7,0 mit 0,3% Detergenzien;
(iii) Zugabe zu dem Gasnzen, das aus Schritt (ii) erhalten wurde, des zu testenden Serums und Inkubation der Mischung während einer genügenden Zeitspanne, um die spezifische Bindung zwischen den Antigenen und Antikörper gegen Trypanosoma Cruzi zu erlauben;
(iv) Auswaschen des Ganzen, das das Serum enthält, mit derselben Pufferlösung, die im Schritt (ii) verwendet wurde;
(v) Herstellung einer Lösung mit Enzymen, die mit den Antigenen gemäß Anspruch 1 konjugiert sind, wobei die genannten Enzyme eine enzymatische Aktivität aufweisen;
(vi) Verdünnung der Lösung mit den in Schritt (v) hergestellten enzymkonjugierten Antigenen #1 und #2, um nichtspezifische Bindungsstellen zu hemmen, wobei die genannte Verdünnungslösung aus Auswaschpuffer mit 5% Rinderserum ist;
(vii) Zugabe der Lösung aus Schritt (vi) zu dem Ganzen aus Schritt (iv) und Inkubation während einer genügenden Zeitspanne, um die Wechelwirkung zwischen den Antikörpern gegen Trypanosoma Cruzi und den Antigenen, die eine enzymatischen Aktivität aufweisen, zu erlauben;
(viii) Entwicklung des Immunkomplexes durch Zugabe der Substratlösung zu der gesamten Mischung aus Schritt (vii) und anschließende Inkubation während einer genügenden Zeitspanne, um die Wechelwirkung zwischen dem Enzym und dem Substrat zu erlauben;
(ix) Hemmung der enzymatischen Aktivität durch Zugabe einer Stoplösung; und
(x) Auswertung der Resultate der enzymatischen Aktivität im Vergleich zu einem Standardserum.

10. Verfahren gemäß Anspruch 9, wobei der Schritt (iii) bei Raumtemperatur zwischen 25°C bis 37°C in einer Zeitspanne zwischen 30 Minuten und 2 Stunden ausgeführt wird.

11. Verfahren gemäß Anspruch 9, wobei der Schritt (vii) bei Raumtemperatur zwischen 25°C bi 37°C und während 30 Minuten ausgeführt wird.

12. Verfahren gemäß Anspruch 9, wobei der Schritt (viii) bei Raumtemperatur zwischen 25°C und 37°C, während 10 Minuten ausgeführt wird.

## Revendications

1. Trousse pour le diagnostic immunologique de la maladie de Chagas, comprenant ce qui suit :
un support en un matériau choisi dans l'ensemble constitué par les polycarbonates, les polyallomères, le polypropylène, le polyvinyle, le nylon et la nitrocellulose ;
et des réactifs, y compris ce qui suit :
(i) un tampon carbonate 50 mM contenant 0,01 à 1,0 mg/ml d'un mélange d'antigène #1 et d'antigène #2 ;
(ii) un tampon de lavage d'une solution salée tamponnée au phosphate (STP), pH 7,0, avec 0,3 % de détergent ;
(iii) une activité enzymatique couplée à l'antigène #1 et l'antigène #2 dans une solution comprenant l'antigène #1 conjugué à une enzyme et l'antigène #2 conjugué à une enzyme pour le développement d'un immunocomplexe ;
(iv) un tampon de dilution constitué du tampon de lavage contenant 5 % de sérum bovin pour la dilution de la solution conjuguée ;
(v) une solution de substrat, pour développer l'activité enzymatique ;
(vi) une solution d'arrêt, pour bloquer l'activité enzymatique ;
(vii) un sérum étalon contenant des sérums positif, négatif et coupé,
l'antigène #1 ayant la séquence d'acides aminés suivante : valine (ou alanine), alanine, acide glutamique, alanine (ou thréonine), acide glutamique, lysine, glutamine, lysine (ou arginine), alanine, alanine, acide glutamique, alanine (ou sérine), thréonine (ou méthionine ou alanine) et lysine, et l'antigène #2 ayant la séquence d'acides aminés suivante : méthionine, acide glutamique, glutamine, acide glutamique, arginine, arginine, glutamine, leucine, leucine, acide glutamique, lysine, acide aspartique, proline, arginine, arginine, asparagine, alanine, lysine, acide glutamique, isoleucine, alanine, alanine, leucine, acide glutamique, acide glutamique, sérine, méthionine, asparagine, alanine, arginine, alanine, glutamine, acide glutamique, leucine, alanine, arginine, acide glutamique, lysine, lysine, leucine, arginine, acide aspartique, arginine, alanine, phénylalanine, leucine, acide aspartique, glutamine, lysine, proline, acide glutamique, arginine, valine, proline, leucine, alanine, acide aspartique, valine, proline, leucine, acide aspartique, acide aspartique, acide aspartique, sérine, acide aspartique, phénylalanine, valine et alanine.

2. Trousse selon la revendication 1, dans laquelle ladite solution comprenant l'antigène #1 conjugué à une enzyme et l'antigène #2 conjugué à une enzyme a été préparée par les étapes comprenant ce qui suit :
(i) le traitement de l'enzyme avec du métaperiodate 0,02 M après la dialyse contre un tampon acétate 1 mM, pH 4,4 ;
(ii) l'addition de l'enzyme traitée au mélange purifié d'antigène #1 et d'antigène #2 ;
(iii) l'addition d'une solution 0,0015 M de carbonate de sodium, pH 9,5, à la solution provenant de l'étape (ii), et l'incubation à la température ambiante pendant 30 à 120 minutes ;
(iv) l'addition d'une solution à 0,2 mg/ml de borohydrate à la solution résultant de l'étape (iii), à 4°C pendant deux heures ; et
(v) la dialyse de la solution résultant de l'étape (iv) contre de la solution salée tamponnée au phosphate, pH 7,2,
l'antigène #1 et l'antigène #2 étant les antigènes indiqués dans la revendication 1.

3. Trousse selon la revendication 1, dans laquelle l'activité enzymatique est choisie dans l'ensemble constitué par la peroxydase, la phosphatase alcaline et l'uréase.

4. Trousse selon la revendication 3, dans laquelle la solution de substrat est un tampon citrate ou phosphate 0,01 M contenant 1 mg de 3',3',5',5'-tétraméthylbenzidine dans 1 ml de DMSO et 0,3 % de peroxyde d'hydrogène, lorsque l'activité enzymatique est la peroxydase.

5. Trousse selon la revendication 1, dans laquelle la solution d'arrêt est une solution 4 M d'acide sulfurique, lorsque l'activité enzymatique est la peroxydase.

6. Trousse selon la revendication 1, dans laquelle le support est une plaque à puits multiples à utiliser dans la technique ELISA directe.

7. Trousse selon la revendication 1, dans laquelle le support est une membrane à utiliser dans l'essai direct en bande.

8. Trousse selon la revendication 1, dans laquelle l'immunocomplexe est développé par l'utilisation de l'antigène #1 et de l'antigène #1 (tels qu'indiqués dans la revendication 1) conjugués à l'activité enzymatique.

9. Procédé pour le diagnostic immunologique de la maladie de Chagas, comprenant les étapes suivantes :
(i) la sensibilisation d'un support en un matériau choisi dans l'ensemble constitué par les polycarbonates, les polyallomères, le polypropylène, le polyvinyle, le nylon et la nitrocellulose, avec un tampon carbonate 50 mM contenant 0,01 à 1,0 mg/ml d'un mélange des antigènes indiqués dans la revendication 1 ;
(ii) le lavage du support sensibilisé avec un tampon de lavage d'une solution salée tamponnée au phosphate (STP), pH 7,0, avec 0,3 % de détergent ;
(iii) l'addition à l'ensemble de l'étape (ii) du sérum devant être testé et l'incubation de l'ensemble pendant un laps de temps suffisant pour permettre la fixation spécifique entre les antigènes et les anticorps dirigés contre *Trypanosoma cruzi* ;
(iv) le lavage de l'ensemble qui contient le sérum avec la même solution tampon que celle utilisée dans l'étape (ii) ;
(v) la préparation d'une solution comprenant des enzymes conjugués avec les antigènes indiqués dans la revendication 1, lesdites enzymes ayant une activité enzymatique ;
(vi) la dilution de la solution comprenant l'antigène #1 conjugué à une enzyme et l'antigène #2 conjugué à une enzyme, telle que préparée en (v), pour bloquer des sites de fixation non spécifique, ladite solution de dilution étant constituée par le tampon de lavage qui contient 5 % de sérum bovin ;
(vii) l'addition à l'ensemble de l'étape (iv) de la solution de l'étape (vi) et l'incubation pendant un laps de temps suffisant pour permettre l'interaction entre les anticorps dirigés contre *Trypanosoma cruzi* et l'antigènes ayant une activité enzymatique ;
(viii) la réalisation du développement de l'immunocomplexe par l'addition à l'ensemble de l'étape (vii) de la solution de substrat et ensuite l'incubation pendant un laps de temps suffisant pour permettre l'interaction entre l'enzyme et le substrat ; (ix) le blocage de l'activité enzymatique par addition d'une solution d'arrêt ; et
(x) l'évaluation des résultats de l'activité enzymatique par rapport à un sérum étalon.

10. Procédé selon la revendication 9, dans lequel l'étape (iii) est réalisée à la température ambiante, entre 25°C et 37°C, en un laps de temps allant de 30 minutes à 2 heures.

11. Procédé selon la revendication 9, dans lequel l'étape (vii) est réalisée à la température ambiante, entre 25°C et 37°C, pendant 30 minutes.

12. Procédé selon la revendication 9, dans lequel l'étape (viii) est réalisée à la température ambiante, entre 25°C et 37°C, pendant 10 minutes.
